# EUROPEAN PATENT SPECIFICATION

(11) **EP 1 877 573 B1**
(45) Date of publication and mention of the grant of the patent: **05.06.2013**
(21) Application number: 06744687.2
(22) Date of filing: 26.04.2006
(51) Int. Cl.: C12Q 1/24, C12M 1/12, C12M 1/20

(54) **METHOD FOR THE DETECTION AND CHARACTERIZATION OF MICROORGANISMS ON A FILTER**
VERFAHREN ZUM NACHWEIS UND ZUR CHARAKTERISIERUNG VON MIKROORGANISMEN AUF EINEM FILTER
PROCEDE DE DETECTION ET DE CARACTERISATION DE MICRO-ORGANISMES SUR UN FILTRE

(30) Priority: 29.04.2005 FR 0504379
(43) Date of publication of application: 16.01.2008
(73) Proprietor: EMD Millipore Corporation, Billerica, MA 01821 (US)
(72) Inventor: MARC, Frédéric, F-67140 Itterswiller (FR); OHRESSER, Serge, F-67190 Still (FR)
(74) Representative: Caen, Thierry Alain
(86) International application number: PCT/IB2006/001230
(87) International publication number: WO 2006/117676

(56) References cited:
- EP-A- 0 439 208
- WO-A-00/28082
- WO-A-01/59157
- WO-A-90/13624
- WO-A-93/19199
- WO-A-99/60005
- WO-A1-94/21780
- US-A1- 2003 057 148
- DATABASE NCBI 7 July 2004 (2004-07-07), XP002402030 Database accession no. AY657857
- ONODERA KENJI ET AL: "Nylon membrane-immobilized PCR for detection of bovine viruses" BIOTECHNIQUES, vol. 32, no. 1, January 2002 (2002-01), pages 74-80, XP001207739 ISSN: 0736-6205
- JOTHIKUMAR N ET AL: "Short communication: Membrane-impregnated probe for simultaneous PCR amplification and detection" WORLD JOURNAL OF MICROBIOLOGY AND BIOTECHNOLOGY, vol. 14, no. 6, November 1998 (1998-11), pages 933-934, XP002402025 ISSN: 0959-3993
- RUIZ ALBERTO ET AL: "Direct RT-PCR amplification of mRNA supported on membranes" BIOTECHNIQUES, vol. 15, no. 5, 1993, pages 882, 884-886, XP009056969 ISSN: 0736-6205
- SCHAAD N W ET AL: "Use of membrane BIO-PCR for ultrasensitive detection of Pseudomonas syringae pv. phaseolicola" PHYTOPATHOLOGY, vol. 86, no. 11 SUPPL., 1996, page S77, XP009056865 & ANNUAL MEETING OF THE AMERICAN PHYTOPATHOLOGICAL SOCIETY, NORTH CENTRAL DIVISION; INDIANAPOLIS, INDIANA, USA; JULY 27-31, 1996 ISSN: 0031-949X

## Description

The present invention relates to a method for the specific detection on a filter of one or more microorganisms present in a fluid, comprising the step of specific isothermal amplification of the nucleic acids of said microorganisms, as well as to a detection kit and sequences for the implementation of this method.

Nowadays the monitoring of the microbiological quality of water and air as well as used or disposed-of tools and materials, within the scope of industrial or medical activities, is subject to increasingly strict standards.

For this reason, industrial and health authorities must be able to have tools allowing them to detect microbiological contamination as early as possible in order to be able to remedy it as soon as possible and at low cost.

Traditionally, microbiological analysis is carried out on agar culture media. These types of cultures, easy to implement, allow germs to be counted with the naked eye and the microorganisms to be kept alive, also allowing characterization tests to be carried out, if appropriate.

These characterization tests generally consist of extracting the nucleic acids contained in the microorganisms and specifically amplifying said nucleic acids, by one of a number of chain reaction techniques (PCR: polymerase chain reaction or LCR: ligase chain reaction) known to a person skilled in the art.

However, although the characterization tests by amplification of nucleic acids are very reliable, the steps consisting of culturing the microorganisms, extracting the nucleic acids there from, and implementing one of the chain reaction techniques described above are time consuming.

To be visible to the naked eye, the microorganisms must be left in culture for at least 24 hours. It is sometimes longer for microorganisms that grow more slowly, such as mycobacteria or for microorganisms that have been stressed by environmental conditions.

Furthermore, the extraction of nucleic acids, which cannot be carried out directly on the agar medium, requires the microorganisms to be sampled prior to their characterization.

PCR, which consists of numerous amplification cycles at different temperatures, has eventually the drawback that it must be carried out in solution in tubes of small dimension which are placed in a thermocycler, which entails the additional work of transferring the nucleic acids before and after amplification.

The recent development of lab-on-a-chip facilities, using different techniques of hybridization with nucleic acid probes fixed on a solid support, could allow time to be saved at the step of characterizing the microorganisms. However, these techniques have an even higher production cost and do not avoid the steps of extraction and transfer of the nucleic acids prior to their characterization.

Under these conditions, more than 24 hours must still be allowed for the microorganisms to be counted and characterized, which is far too long in an industrial context where a permanent monitoring of the quality of the products must be ensured.

In the particular field of the quality control of industrial water intended for the composition of injectable pharmaceutical solutions, for instance, information on the quality of the water introduced into the production chain must be able to be obtained virtually in real time.

The detection of the microorganisms must take place *in situ* and, if possible, with a minimum of equipment and staff.

Implementing detection is all the more difficult in this context that the desired level of detection is of the order of only a few microorganisms per litre of water, which requires a prior step of concentrating the microorganisms by filtration.

To respond to these limitations, an approach for the rapid determination of the presence or absence of microorganisms has been first proposed by the applicant in the international application WO 01/59157.

This approach consists of reducing culture duration by basing the detection of the microorganisms on their metabolic activity.

A universal metabolic marker, which for example can be ATP contained in the microorganisms, or also the emission of CO₂, is measured, thus establishing the presence of the microorganisms before colonies are visible to the naked eye on the agar medium.

In the example of the microbiological monitoring of water, a certain volume of water (comprised between 100 ml and 1l) is filtered through a filtering membrane so as to trap the microorganisms contained in the water. The filtering membrane is then placed on an agar medium, which is exploited by the microorganisms for initiating growth. Then, the membrane is recovered to detect the ATP of the microorganisms present on its surface, by reacting ATP with an enzyme (Luciferase) producing photons. The luminescence is measured using an image analysis system.

This system, marketed by the applicant under the name Milliflex® Rapid system, has already reduced the time necessary to establish the presence or absence of microorganisms in a liquid by several hours.

This system produces quantitative, but not qualitative, information. When the nature of the microorganisms present has to be established, or a given microorganism is specifically sought, a step of characterization, generally carried out by PCR, must be added which, as previously mentioned, significantly increases the duration of the test.

International patent application WO 1993/19199 discloses another device for identifying microorganisms comprising a filter in contact with a grid forming a plurality of wells and with a base member connected to a vacuum pump. However, the filter must be removed from the device for incubation, and thus the device must be opened and dismantled to carry out this step.

International patent application WO 1994/21780 discloses a method for detecting and numerating microorganisms present in a liquid sample. The samples are filtered, the cells fixed on the filter by heating and detection is carried out by PCR.

Meanwhile, a technique for the specific amplification of nucleic acids has been developed under the name LAMP (loop-mediated isothermal amplification) the principle of which is described in the International Applications WO 00/28082, WO 01/077317 and WO 02/024902.

Unlike PCR, this method has the feature of being carried out at constant temperature (isothermal condition) while maintaining a high level of specificity.

It is an object of the present invention to provide a method obviating the drawbacks of earlier detection methods consisting in implementing the detection directly on a filter by amplification of their nucleic acids.

In a surprising manner, an isothermal amplification technique, such as the LAMP technique, usually implemented in solution after culture and extraction of nucleic acids, can be performed on a filter where the microorganisms have been trapped, without necessarily resorting to the step of culturing the microorganisms and transferring their nucleic acids to a tube for amplification.

The method of the invention therefore allows the microorganisms present in a volume of water, gas or on the surface of a solid, to be detected over a reduced time period in a specific manner with a detection threshold of only a few microorganisms.

The invention allows individual counting *in situ* on a membrane, i.e. in a given part of the membrane, of the microorganisms trapped thereon.

The present invention therefore relates to a method for the specific detection on a membrane of one or more microorganisms present in a fluid, characterized in that it comprises the following steps:
a) the liquid, the gas or the surface is put in contact with the membrane so as to trap the microorganism or microorganisms on the membrane;
b) said microorganisms are treated on said membrane with a fixation solution containing a cross-linker selected from glutaraldehyde, formaldehyde and/or paraformaldehyde;
c) the nucleic acids present in the microorganism or microorganisms are specifically isothermally amplified on the membrane in order to obtain amplification products;
d) the obtained amplification products are detected.

The invention also relates to a device, a kit and primers specifically suitable for the implementation of the method according to the invention.

The aim of the figures below is to illustrate certain features of the invention:
**FIGURE 1****:** Principle of the LAMP amplification technique suitable for the detection of messenger RNAs contained in the microorganism or microorganisms, preferably used in step c) of the detection method according to the invention.
**FIGURE 2****:** DNA sequence originating from the *oprL* gene (5' -> 3') showing the primers used for the specific detection of *Pseudomonas aeruginosa* by the LAMP technique according to the method of the invention. The sequences corresponding to the primers used according to the invention are shaded and their orientation is indicated by the arrows.

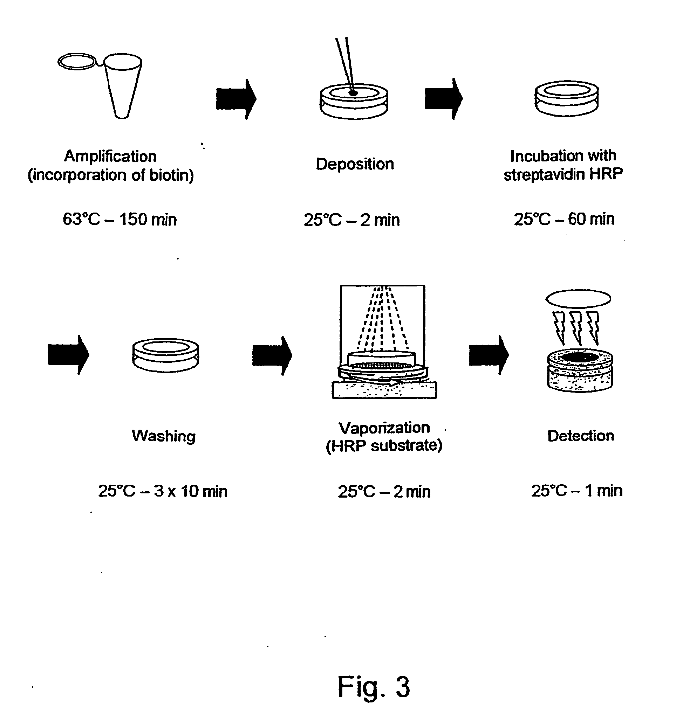

**FIGURE 3****:** Steps of the method according to the invention, implemented in Example 1.
**FIGURE 4****:** Illustrations of steps c) and d) of the method according to the invention, described in Example 1
**FIGURE 5**: Steps of the detection method according to the invention, implemented in Example 2.
**FIGURE 6****:** Steps c) and d) of the detection method according to the invention, described in Example 2.

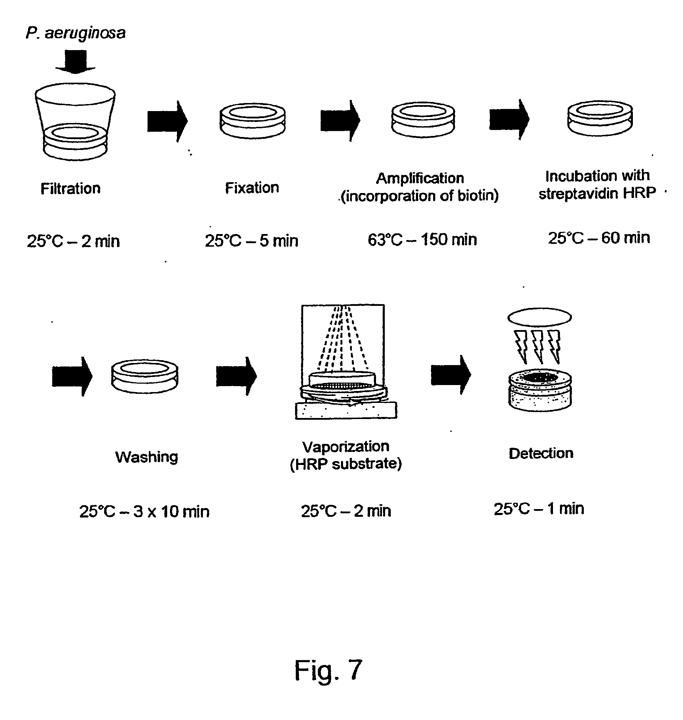

**FIGURE 7**: Steps of the detection method according to the invention, implemented in Example 3.

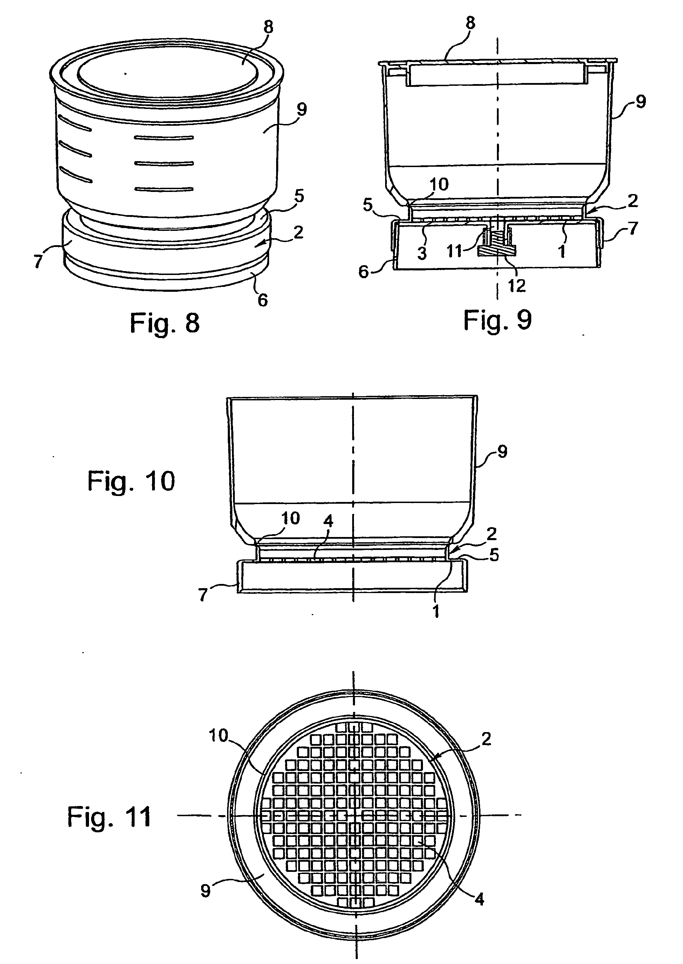

**FIGURE 8****:** Perspective view of a device for the amplification of nucleic acids intended to implement the method according to the invention.
**FIGURE 9****:** Cross-section of the device in Figure 8.
**FIGURE 10****:** Cross-section of the device in Figure 8 without its flat wall and its cover.
**FIGURE 11**: Top view of the figure of the device in Figure 10.

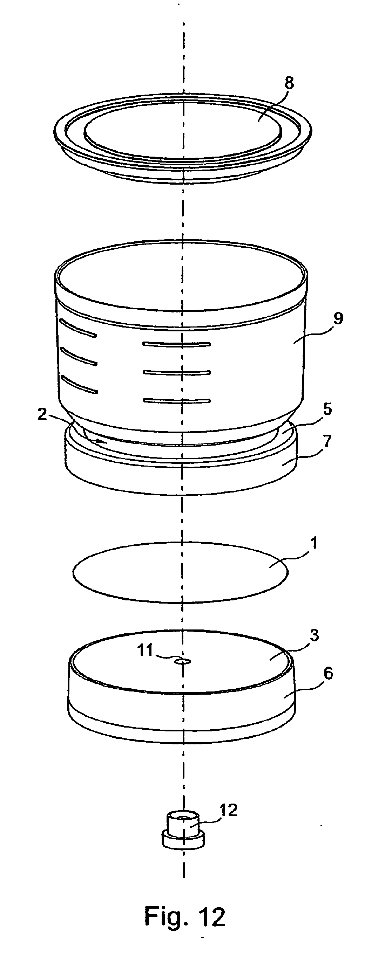

**FIGURE 12****:** Perspective exploded view of the device in Figure 8.
**FIGURE 13****:** Perspective view of the device in Figure 8 after its reservoir has been removed.
**FIGURE 14****:** Transverse cutaway view of the device in Figure 13.
**FIGURE 15**: Perspective exploded view of the device in Figure 13.

### Detailed description of the invention:

The detection method according to the invention allows the monitoring of the microbiological quality of a fluid, such as a liquid or a gas, but also of a surface.

This method is thus applicable to a large number of both industrial and health applications, such as for example the monitoring of water, during production and retreatment in agri-food or pharmaceutical contexts, of air emitted from air-conditioning systems or inside clean rooms, or the monitoring of the asepsis level of work surfaces in laboratories, factories and operating theatres.

This method can also be used for diagnostic purposes, for example for the analysis of urine in the case of urinary infection, cerebrospinal fluid in the case of meningitis, amniotic fluid in the case of foetal infections or abnormalities, plasma for viral infection research, or exhaled air in the case of bronchial or pulmonary infection.

More generally, it constitutes an alternative to the lab-on-a-chip for the characterization of nucleic acids, regardless of the envisaged application.

The microorganisms aimed at by the method of the invention are in general chosen from the pathogenic bacteria of the genera *Pseudomonas, Escherichia, Legionella, Salmonella, Listeria, Bacillus, Streptococcus, Vibrio, Yersinia, Staphylococcus, Mycobacterium, Shigella, Clostridium, Campylobacter,* or *Aeromonas;* protozoa of the genera *Giardia, Entamoeba, Cryptosporidium, Cyclospora;* mycoplasmas of the genera *Mycoplasma* and *Ureaplasma,* fungi of the genus *Aspergillus, Candida* or *Penicillium,* the hepatitis A and E viruses, which are pathogenic contaminants present in the environment. This list is not meant to be limiting but merely to identify the organisms of most interest in the industry. Other organisms may also be of interest and it is meant to include them within the present invention.

In the sense of the present application, the term "microorganism" is not restricted to bacteria, fungi and yeast. It also applies to other microscopic living forms like unicellular algae such as cyanobacteria, unicellular forms of parasitic organisms, such as amoeba or nematode eggs, or also plant or animal gametes such as pollens or spermatozoa, and even microscopic pluricellular organisms like trematodes or ascaris.

According to one aspect of the invention the microorganisms are initially present in a liquid, a gas or on a surface.

A liquid in the meaning of the invention may be a solution which can comprise a solid phase in suspension, more preferentially a solution in aqueous medium. A gas is preferably constituted by a mixture of gas such as air, but can also by extension consist of a mixture of solid, liquid and gas particles, such as an aerosol.

A surface is preferably that of a solid such as for example the floor or the walls of a clean room.

The microorganisms are trapped, i.e. retained, on a filter or in the depth of said filter, either by filtration or by contacting said filter with a contaminated surface.By "filter" is meant, in the sense of the present invention, a medium of which the main constituent is a material, which, by its nature, its size and/or its arrangement, retains the microorganisms contained in suspension in a liquid or gaseous flow passing through it.

The term "filter" thus designates various types of filtering items such as filtration pads, membranes or microcolumns filled with chromatography media such as microbeads, gels or resins.

According to the present invention, the filter is a membrane.

According to a preferred embodiment, the material which is the main constituent of the filter is coated with antibodies or chemical groups, which interact with the outer membrane of the microorganisms or with the nucleic acids therefrom to help trap or affix the organisms to the filter.

The filter can be mono- or multi-layered. In general it is constituted by one or more materials chosen from glass, latex, polytetrafluoroethylene, poly(vinylidene) fluoride, polycarbonate, polystyrene, polyamide, polysulphone, polyethersulfone, acetyl cellulose, mixed celluloses and nitrocellulose, or any materials known by one skilled in the art in the field of filtration items.

The pores of the membrane in general have an average diameter comprised between 0.1 and 1.5 microns, preferably between 0.15 and 0.8 microns, more preferentially between 0.2 and 0.6 microns.

The materials are preferably chosen to be compatible with the solvents used during steps b), c) and d) of the method, in particular alcohols and aldehydes capable of being used to fix the microorganisms on the filter and release the nucleic acids contained in these microorganisms.

By "membrane" is meant in the present Application a filter constituted by a synthetic support having two surfaces, the pores of which have an average known diameter. This average known diameter is calculated so as to prevent at least 99 % of the microorganisms having the dimension of said average diameter from passing through the membrane. The membrane used within the scope of the present invention in general has a high surface/volume ratio and a constant thickness preferably comprised between 90 and 200 microns.

A membrane according to the invention is preferably a PVDF filtering membrane, such as for example the membrane marketed by Millipore Corporation under the brand name MXHVWP124.

According to a preferred aspect of the invention, the microorganism or microorganisms are trapped on the membrane by filtration, which is carried out by passing a liquid or a gas through the pores of the membrane by applying a pressure difference between the two surfaces of the membrane. The microorganism or microorganisms are then retained by the surface and/or pores of the membrane.

According to a preferred feature of the invention, step a) of the method consists of a filtration which aims to concentrate the microorganisms present in the liquid or the gas on the membrane.

When attempting to retain yeast- and mold-type microorganisms, it is advisable to choose an average pore diameter comprised between 0.5 and 1.5 microns, preferably between 0.6 and 1 micron. For bacterial microorganisms, an average diameter comprised between 0.2 and 0.8 micron, preferably between 0.3 and 0.5 micron, is chosen. For mycoplasma-type microorganisms and viruses, a diameter of less than 0.2 micron, preferably between 0.1 and 0.2 micron, is chosen. The pore size is obtained by statistical calculation since in general the pores result from the superposition of different layers of the same material or different materials each with an irregular porosity.

It can be advantageous during filtration to use a filtering membrane comprising a hydrophobic-type mesh forming a network of a solution of hydrocarbonated, non-wettable waxes, vaseline, silicone waxes or oils, epoxy resins, polyfluoroethylene or polystyrene, so as to better redistribute the microorganisms over the whole membrane and to be able to count them better in step d).

In the absence of filtration, the microorganisms are trapped by simply putting the microorganism in contact with the surface of the filter. This contact can be produced by active projection of a liquid or a gas in the direction of a filter (air flow, water current) or passively when the microorganism is deposited by gravity on the filter. Conversely, the filter can be applied to a solid surface on which the microorganism is located. For this trapping method, it can be advantageous for the surface of the filter to be covered with a coating facilitating the adhesion of the microorganisms. This coating can be made of glycerol for example.

If desired, a step of culturing the microorganism or microorganisms immobilized on the filter can be carried out before amplification step c). Such a culturing can take place directly on the filter like a membrane by putting this one in contact with a nutritive medium, in particular an agar medium, which if appropriate allows the number of microorganisms present to be increased, while respecting the initial location of the microorganism or microorganisms on the filter. The colonies obtained can serve as biological materials for step d), or can be sampled with a view to other analyses, such as for example quantification by ATP bioluminescence.

The nucleic acids contained in the microorganism or microorganisms trapped on or in the depth of the filter consist essentially of double- or single-stranded DNA and RNA. These can consist of in particular chromosomic DNA, chloroplastic DNA, mitochondrial DNA, ribosomic RNAs, transfer RNAs or messenger RNAs.

According to a preferred mode of the invention, the external wall of the microorganisms is lysed before amplification step d) in order to release the nucleic acids contained in the microorganisms. The lysis step can be carried out directly on the filter, for example, by impregnating the latter with a lysis buffer. It can be completed if appropriate by a purification step consisting, for example, of precipitating the nucleic acids with alcohol on the surface of or inside the filter.

The microorganisms trapped on the filter with their nucleic acids are fixed on the filter by an appropriate treatment. This fixation step b) prevents the migration and mixing of nucleic acids originating from the different microorganisms. This treatment can consist of the impregnation of the filter by a fixing solution comprising, for example, a cross-linker such as glutaraldehyde, formaldehyde or paraformaldehyde. Glutaraldehyde is particularly advantageous when a polyamide-based membrane is used.

According to step c) of the method according to the invention, the nucleic acids present in the microorganism(s) are specifically amplified on the filter in isothermal manner in order to obtain amplification products.

By specific amplification is meant here a polymerization reaction of nucleic acids initiated by the hybridization of a specific primer complementary to a nucleic acid serving as template, with a view to reproducing all or part of the sequences comprising this nucleic acid serving as template.

Unlike PCR techniques, the amplification step c) according to the invention does not require cyclic changes of temperature, and therefore requires neither the use of a thermocycler, nor consumables of small dimension which are associated with it, designed to ensure an effective heat transfer to the reaction medium.

The amplification step is carried out on a filter, i.e. on the surface of or inside the latter, and more preferentially in an enclosure at controlled temperature. In general, the temperature is maintained between 35 and 90°C, preferentially between 50 and 70°C, and more preferentially between 60 and 65°C.

Different methods for the isothermal amplification of the nucleic acids can be applied to the method of the invention such as, for example, the NASBA technique (nucleic acid sequence based amplification), or TMA (transcription mediated amplification), which allows single-stranded RNA amplicons to be obtained [Nature, 1991, 350: 91-92].

A preferred amplification technique according to the invention is the LAMP technique by which a DNA template is reproduced via amplicons forming stem loops.

The principle of the LAMP technique is described in the International Application WO 00/28082.

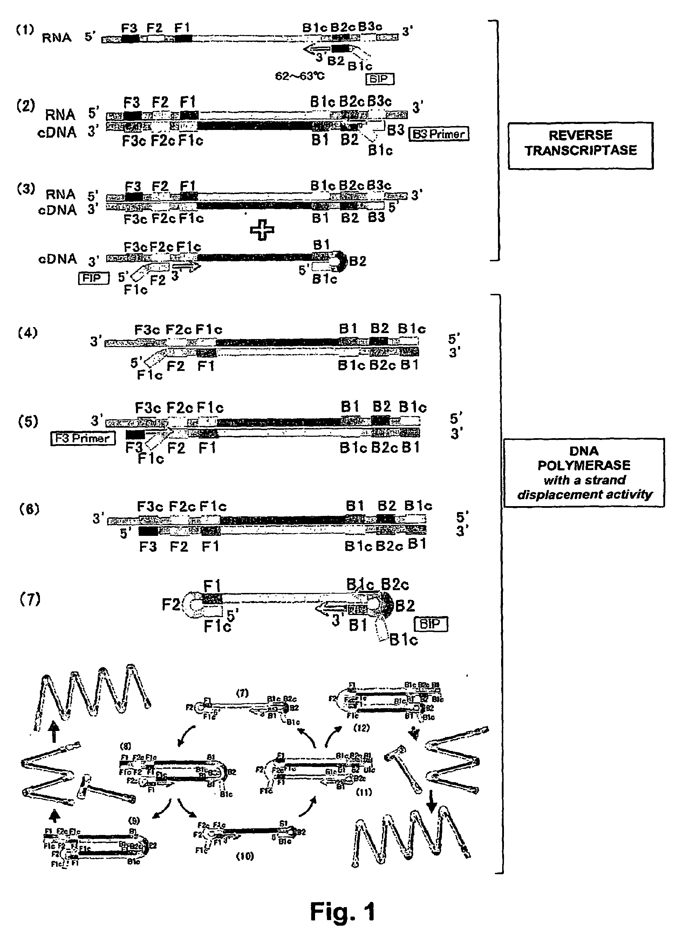

A preferred adaptation of this technique according to the invention is illustrated in Figure 1.

By LAMP-type amplification technique is meant any isothermal amplification technique derived from the LAMP technique, comprising at least the step by which a DNA polymerase, having a strand-displacement activity, synthesizes from a template of nucleic acids a strand of DNA complementary to the latter via a specific "FIP" primer (Forward Inner Primer). The FIP primer is characterized in that it hybridizes to the template at its 5' part, but not at its 3' part. In fact its 3' part has a sequence which can subsequently hybridize with one of the sequences of the complementary strand formed. The result is that, once the complementary strand has synthesized, the 3' part of FIP will favour hybridization on itself of the synthesized strand, creating a stem loop structure. This stem loop structure is then reproduced in the subsequent steps of the amplification.

The LAMP-type amplification preferentially requires a second "BIP" primer (Backward Inner Primer) which has the same function as FIP, but on the opposite strand. More preferentially, the complementary primers called F1, F2, F3 (F: Forward) and B1, B2. B3 (B: Backward) are used. The F1, F2, F3 primers correspond to sequences situated on the coding strand and the B1, B2, B3 primers to sequences on the complementary strand. F3 and B3 correspond to the 5' ends of the amplified fragments, F2 and B2 correspond to the sequences situated at the 5' end of FIP and BIP, F1 and B1 correspond to the complementary sequences of the sequences situated at the 3' end of FIP and BIP (called F1c and B1c).

According to the type of nucleic acids to be amplified, a phase of denaturation of the DNA by heating to between 60° and 80°C aimed at separating the DNA strands of the double-stranded DNA molecules may be necessary at the start of the amplification procedure.

In general, a LAMP-type amplification technique uses a single- or double-stranded DNA as starting template.

According to a preferred aspect of the method according to the invention, the starting template is a cDNA.

According to a more preferred feature of the invention, the RNAs of the microorganisms are retro-transcribed in advance with the help of a reverse transcriptase using a BIP primer, as indicated in Figure 1, which allows a cDNA comprising a stem loop structure to be obtained, allowing the LAMP-type amplification to be started directly.

According to an even more preferred feature of the invention, the RNAs originating from the microorganisms used for the previous retro-transcription step are messenger RNAs. In fact, messenger RNAs have the advantage of being present in much greater numbers than the DNA molecules serving for their transcription. In this way it is possible to lower the detection threshold of the method according to the invention.

The LAMP-type amplification technique advantageously comprises a phase during which additional primers loop F and loop B hybridize to the amplification products at the level of the sequences constituting the loops. These primers promote the synthesis of new complementary strands starting from the loops that comprise the amplification products, which allows the speed of formation of the amplification products to be increased.

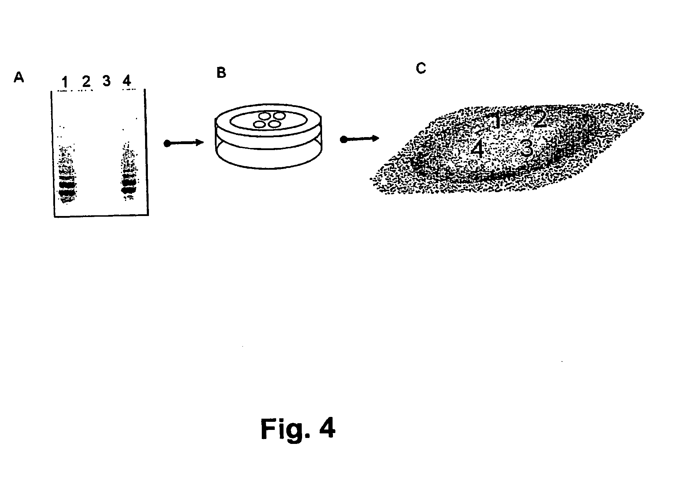

The amplification products obtained according to a LAMP-type technique are generally double-stranded DNA molecules comprising several stem loops. The size of the amplification products varies as a function of the number of loops synthesized. In general, several sizes of fragments co-exist at the end of the amplification method, which results in an electrophoretic profile of the type represented in Figure 4.

According to a preferred feature of the invention, step d) of the detection of the amplification products is carried out by methods directly or indirectly using the marked molecules, allowing detection by fluorescence, colorimetry or chemoluminescence of the amplification products.

An image analyzer can then be used to record the emission spectrum of the marked molecules and the position of the latter on the filter. By appropriate treatment of the signal, aimed in particular at eliminating the parasitic signals linked to the non-specific amplifications, it is possible to view the amplification products *in situ.* This is particularly useful when a membrane is used, because the large surface of the membrane allows quantitative and qualitative precision.

The means for detecting the amplification products by colorimetry or fluorescence are known to a person skilled in the art.

Preferably, the detection means used according to the method of the invention are specific and allow the individual identification of the microorganism or microorganisms the nucleic acids of which have been amplified.

A detection means can consist, for example, of the incorporation of modified purine or pyrimidine bases into the amplification products during step b) of the method, as substrate of the amplification reaction. Preferably, the marked purine or pyrimidine bases are dNTPs, more preferentially dUTPs.

Another detection means can consist of hybridizing at the end of step c) the amplification products obtained with one or more marked specific probes. Different types of probes can be used, such as, for example, PNA-type probes (Peptide Nucleic Acid), i.e. probes constituted by a polypeptide chain substituted by purine and pyrimidine bases, the spatial structure of which mimics that of a DNA [Nielsen, P.E. et al. (1991) Sequence selective recognition of DNA by strand displacement with a thymine-substituted polyamide, Science 254:1497-1500].

The probes used are advantageously of the "molecular beacon" type, i.e. they have a fluorophore, a quencher group and a stem loop structure allowing a fluorescence of the probe only when it is hybridized to the amplification products.

When the amplification products are detected using hybridization probes, it may prove necessary to proceed to a preliminary step of denaturation of the amplification products, aimed in particular at separating the DNA strands.

Another detection means according to the method of the invention consists of the use of specific ligands, preferably marked antigens and antibodies. In this case, modified purine or pyrimidine bases, or even specific probes, are marked using one or more molecules serving as antigens, such as dioxygenin, being able to be specifically recognized by an antibody such as for example an anti-dioxygenin antibody. Alternatively, the detection probes can be coupled to an antibody which is then put in contact with a marked antigen. The antibodies or antigens can be conjugated to a fluorophore, such as FITC (a particular form of isothiocyanate).

The marking of the modified purine or pyrimidine bases, or of the specific probes can be carried out by radioactive isotopes (H³, P³², P³³, S³⁵...) or by non-radioactive products such as, for example, biotin, which is detected by adding a solution containing conjugated avidin or streptavidin to an enzyme such as Raifort peroxidase or soya seed or alkaline phosphatase. When a substrate of these enzymes is spread, for instance on the surface of the membrane, these enzymes release photons, which causes a detectable luminescent reaction.

A preferred method of marking according to the invention consists of coupling the hybridization or the ligands mentioned above to fluorescent molecules or fluorophores. Fluorophores having different emission spectra can advantageously be used during the detection step d), coupled to the ligands or probes defined to recognize the amplification products originating from different microorganisms. To this end, fluorophores such as those marketed under the name ALEXA FLUORINE^{®} by Molecular Probes or DY-^{®} by Dyomics are advantageously used, since they have different emission spectra. A parallel detection carried out on different species of microorganisms is consequently possible. After appropriate image processing, a color code can be attributed to each of them which allows the different microorganisms or colonies of each sought species to be determined. This provides a particularly good image when it is performed on a membrane as a filter medium.

It is also possible to mark the probes or the ligands used in step d) with very fine particles known as "quantum dots" and marketed under the name Qdots®. These are inorganic fluorophores composed of nanocrystals the fluorescence of which is much more stable over time. Their use is particularly suited to the detection of less abundant targets requiring a longer time for collection of the data by an image analyzer. The "quantum dots" are also compatible with a multi-coloured marking; they therefore allow the simultaneous detection of different species of microorganisms.

Within the scope of the present invention, when using a membrane as a filter, it is preferable to use dyes which have a fluorescence in the near infrared, such as IRdyes marketed by LiCor^{®} under the names IRdye 700 and IRDye 800 or marketed by Dyomics^{®} for example under the names DY-700, DY-730, DY-750 and DY-780, in order to avoid the fluorescence due to the synthetic materials which compose the membrane. It was in fact observed that the membrane generally emitted less fluorescence in the near infrared.

The method according to the invention aims at detecting in a gas, a liquid or on a surface fewer than 1000 microorganisms of the same species, preferentially fewer than 50 microorganisms of the same species, more preferentially, lower than 5 microorganisms from the same species and down to just one microorganism of a given species.

In order to avoid the displacement of the microorganisms or their nucleic acids on the surface of the filter during one of the steps of the method described above, it is advantageous to use means which limit the exchanges, which can operate via the reaction solutions, between the different zones on said surface.

One means of limiting these exchanges consists for example of putting the reaction solution in contact with the microorganisms or nucleic acids via a pad impregnated with the reaction solution. The impregnated pad, when it is plated, for instance, on the surface of a membrane, delivers locally a volume of reaction solution sufficient for the reaction to be able to take place, without overflowing towards the adjacent zones.

Another means of limiting the exchanges can consist of formulating the reaction solution in the form of a gel, preferably an acrylamide gel.

Another means of limiting the exchanges according to the invention consists of a vertical partitioning positioned on the filter delimiting different zones of the latter. Advantageously, this partitioning is designed to provide a certain volume on the surface of a membrane so as to accommodate the reaction solution. Preferably, the partitioning is presented in the form of a grid forming a plurality of cells which is kept in contact with the filter.

According to a preferred feature of the invention, the present method applies to the specific detection on a filter of one or more bacteria of the species *Pseudomonas aeruginosa* contained in a liquid, characterized in that it comprises the following steps:
a) the liquid is passed through a membrane;
b) the bacteria contained in the liquid are trapped on the membrane;
c) said bacteria are treated on said membrane with a fixation solution containing a cross-linker selected from glutaraldehyde, formaldehyde and/or paraformaldehyde;
d) nucleic acids of *Pseudomonas aeruginosa* contained in these bacteria are specifically isothermally amplified on said membrane with the help of a LAMP-type amplification by using at least one primer comprising a sequence taken from SEQ ID No.2, SEQ ID No.3, SEQ ID No.4, SEQ ID No.5, SEQ ID No.6, and SEQ ID No.7;
e) the amplification products obtained on the membrane are detected.

According to a particularly preferred feature of the invention, the primers used for the amplification of the nucleic acids of *Pseudomonas aeruginosa* by the LAMP-type technique are as follows:
- F3 : CCTCCAAGGGCGGCGATGC (SEQ ID No.2)
- B3 : TGCCTTTCAGGTCTTTCGCGTGT (SEQ ID NO.3)
- FIP : CTGCCGTCAACGGCACCGCTTTTCCGGTGAAGGTGCCAATGGC (SEQ ID NO.4)
- BIP : GCGTGCGATCACCACCTTCTACTTTTCAGAGCGCGCATGGCTTCC (SEQ ID No.5)
- LOOP F: CCTGCGTTCGGGTCGACGC (SEQ ID NO.6)
- LOOP B: CGAGTACGACAGCTCCGACC (SEQ ID No.7)
These primers allow the amplification of the region of *Pseudomonas aeruginosa* represented by SEQ ID No.1 (region of the *oprL* gene).

The messenger RNAs contained in *Pseudomonas aeruginosa* are preferably retro-transcribed in Step b) in order to obtain a cDNA comprising the sequence SEQ ID N°1, which is then amplified according to the LAMP-type amplification technique.

Another feature of the invention relates to a device for the amplification of the nucleic acids, suitable for implementing the method according to the invention described previously. This device, which contains a filter (1) for receiving the nucleic acids to be amplified is characterized in that it contains a body (2) delimiting an internal volume and provided with means for keeping the filter (1) in contact, in this internal volume, with a flat impermeable wall (3) and with a grid (4) forming a plurality of cells on the surface of the filter (1).

Preferably, the cells, which can be of variable size and geometry, are designed such that they make it possible to contain a volume, generally comprised between 1 and 100 µl, preferentially between 1 and 50 µl and more preferentially between 3 and 10 µl, of a reaction solution.

The body (2) of the device can have a shoulder (5) suitable for cooperating with the periphery of the filter (1), such that the latter can be fixed by its periphery against said shoulder (5).

The flat wall (3) can also serve to secure the periphery of the porous filter (1) against said shoulder (5). It can be detachable vis-à-vis the body and, moreover, have a skirt (6) extending transversely. In this case, the body (2) advantageously has a sleeve (7) intended to cooperate with the skirt (6), which can have a shape opening out away from the flat wall (3).

The grid (4) can be independent of the body or in a single piece with the body (2). When it is independent of the body, it can be recessed into the body, such that the partitions of the cells are uniformly in contact with the surface of the filter. The cells are preferably of equal size and regular square or parallelepiped shape in order to be able to be filled with an equal volume of a reaction solution. When the grid and the body form just one piece, the filter can be then advantageously sealed on the grid in order to avoid cross-contaminations between cells.

According to another feature of the invention, the device has a reservoir (9) attached to the body (2). This reservoir is intended to be filled with a liquid that is filtered by passage through the filter (1) (which is then a filtering filter), for example with the help of a vacuum pump which is fitted to the sleeve (7) in place of the flat wall (3). The microorganisms contained in the liquid are then trapped on the admission or upstream side of the filter. Once this operation has finished, the flat wall (3) is replaced in hermetic manner against the lower surface or downstream face of the filter.

To avoid contamination of the liquid before filtration, it is envisaged that the reservoir can be provided with a detachable cover (8).

The reservoir (9) can advantageously be attached to the body (2) by a frangible zone (10) such that once the filtration operation is finished, the reservoir can be detached by exerting from top to bottom a strong pressure on the upper horizontal edge of the reservoir.

According to a preferred feature of the invention, the detachable cover (8) is suitable for alternately closing the reservoir (9) and said internal volume at the level of the frangible zone (10).

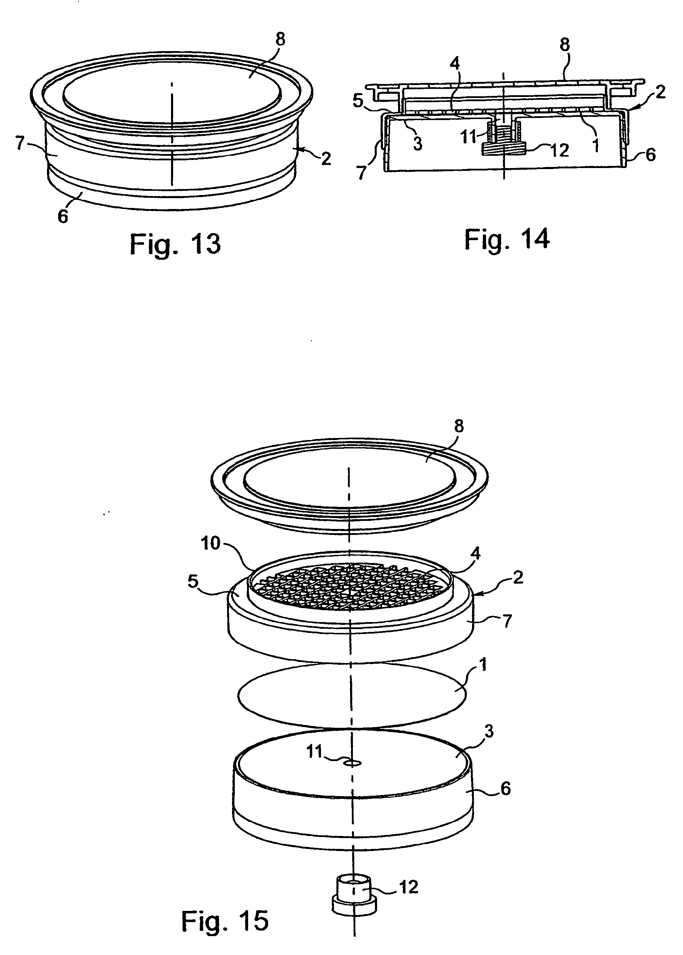

Once the cover has closed on the frangible zone, the detachable cover (8) is then located opposite the grid (4) to close said internal volume, as is shown in Figures 13 and 14. The device in this shape allows the filter to be confined in a volume delimited by the flat wall (3), the body (2) and the cover (8), and vertically partitioned by the grid (4). This volume is intended to receive the reaction solutions for the amplification and optionally other steps of the method, such as the purification, fixing, and washing steps mentioned above. This volume is preferably sealed so as not to lose solution.

The confinement of the filter in a reduced reaction volume is important to be able to realize the amplification reactions in satisfactory conditions. In fact, on the one hand contamination originating from the external environment must be avoided, and on the other hand the enzymatic reactions must not take place in too large volumes. This is necessary because the enzymatic reagents are expensive and the temperature must be regulated in uniform manner. Finally, too great an evaporation of the reaction solutions must be avoided.

The deposition of said solutions is preferably carried out by temporarily removing the cover, applying the solution(s) over the grid, using a micropipette or a vaporizer. The volume of the solution can exceed the height of the grid or not.

According to a preferred aspect of the device according to the invention, the flat wall (3) has a drainage orifice (11), and if appropriate a stopper (12) suitable for closing the drainage orifice (11). It is then possible, after the amplification or marking step according to the method of the invention, for example, to carefully remove the solution without the risk of excessively putting into suspension the nucleic acids located on the filter. Once the amplification and marking steps have been carried out, it is possible to retain only the body of the device provided with the filter, or, if appropriate, the filter alone, in order to analyze the latter according to the chosen technology, such as for example analysis by bioluminescence using an image analyzer.

Another feature of the invention relates to a kit for the implementation of the detection method according to the invention.

This kit comprises at least:
- a membrane;
- a fixing solution containing gluteraldehyde, formaldehyde or paraformaldehyde;
- one or more specific primers allowing amplification of the nucleic acids in isothermal manner.

It may also comprise an amplification solution comprising a polymerase enzyme, a lysis solution, a blocking solution to stop the reactions and a detection solution comprising one of the detecting agents referred to in the present application.

By "specific primers allowing amplification of the nucleic acids in isothermal manner" is meant molecules capable of hybridizing and initiating a polymerization reaction in the presence of a DNA polymerase, such as for example the FIP and BIP oligonucleotides in the case of a LAMP-type amplification, or in the presence of an RNA polymerase as in the case of an NASBA-type amplification.

A kit according to the invention intended more particularly for the detection of *Pseudomonas aeruginosa* bacteria, comprises at least one oligonucleotide comprising a DNA sequence chosen from SEQ ID No.2, SEQ ID No.3, SEQ ID No.4, SEQ ID No.5, SEQ ID No.6 and SEQ ID No.7.

Another feature of the invention relates to an oligonucleotide allowing the specific detection of *Pseudomonas aeruginosa* by amplification of the nucleic acids contained in this bacterium, characterized in that its nucleotide sequence comprises a sequence chosen from SEQ ID No.2, SEQ ID No.3, SEQ ID No.4, SEQ ID No.5, SEQ ID No.6 and SEQ ID No.7.

The examples below aim to illustrate the invention in a more detailed manner.

### Example 1

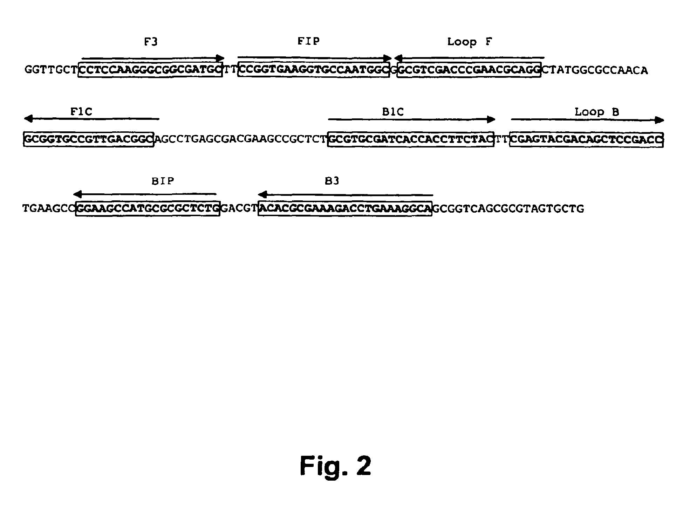

A test illustrated in Figure 4 of the present Application consisted of amplifying according to the LAMP technique illustrated in Figure 1 the messenger RNAs of only 50 *Pseudomonas aeruginosa* cells. The primers used for this purpose are those described in Figure 2 of the present Application.

In order to be able to be precise about the number of cells treated, the nucleic acids were extracted from the bacteria and the amplification carried out in 25µl of the solution (2 pmol/µl of FIP BIP oligonucleotides; 1 pmol/µl of loop F & loop B oligonucleotides; 0.2 pmol/µl of F3 & B3 oligonucleotides in 40 mM Tris HCl pH 8.8, 20 mM KCI, 0.6 M betaine, 1.5 mM each of dNTP + 0.2 mM UTP biotin, 20 mM (NH₄)₂SO₄, 8 mM MgSO₄, 0.1% Triton® X100 surfactant, 0.005 U/µl AMV RTase, 0.32 U/µl *B. st* DNA polymerase) in a tube left in a water bath at 63 °C for 3 hours.

The amplification was carried out under the following conditions:
- tube No. 1: treatment of 50 *P. aeruginosa* cells;
- tube No. 2: control without cells;
- tube No. 3: control without cells + dUTP biotin;
- tube No. 4: treatment of 50 cells + dUTP biotin

The amplification products were observed on agarose gel to ascertain the presence of amplification products in reaction mixtures 1 and 4 and the absence of amplification in mixtures 2 and 3.

The products of the 4 reactions were applied to a nylon membrane.

The membrane was then incubated with a blocking solution (Pierce blocking buffer + 1% SDS) for 45 minutes, then put in contact with a solution of streptavidin conjugated to Raifort peroxidase (1 mg/ml), which was diluted 1:750 in the blocking solution; after 45 minutes of incubation, the membrane was rinsed using a washing solution (PBS with Tween® surfactant, 0.2 % v/v) for 30 minutes. A luminol-based substrate, marketed by Pierce Chemical, of black radish peroxidase was sprayed onto the surface of the membrane in order to allow the luminous reaction to occur.

Point C/ of Figure 4 shows the image obtained after processing of the image by an analyzer (CCD camera). The reaction obtained between the amplification products labelled by the biotin and the peroxidase can easily be seen for reaction mixture No.4.

This experiment shows that amplification products obtained from a quantity of nucleic acids equivalent to 50 cells of a microorganism such as *Pseudomonas aeruginosa* can be easily detected on membrane.

### Example 2

Another test illustrated in Figure 4 consisted of applying ∼ 130 *Pseudomonas aeruginosa* cells to a membrane (MXHVWP124).

After this application, the membrane was placed on a cellulose pad impregnated with 1.5 ml of the fixation solution (0.25% glutaraldehyde in 95% of denatured alcohol) for 5 minutes at ambient temperature then dried for 5 minutes at ambient temperature.

The membrane was placed in a hermetic Petri dish then 0.5 ml of the amplification solution (2 pmol/µl of FIP & BIP oligonucleotides; 1 pmol/µl of loop F & loop B oligonucleotides; 0.2 pmol/µl of F3 & B3 oligonucleotides in 40 mM Tris HCl pH 8.8, 20 mM KCI, 0.6 M betaine, 1.5 mM each of dNTP + 0.2 mM UTP biotin, 20 mM (NH₄)₂SO₄, 8 mM MgSO₄, 0.1% Triton® X100 surfactant, 0.005 U/µl AMV RTase, 0.32 U/µl DNA polymerase) was added before incubation at 63°C for 150 minutes.

The membrane was then incubated with a blocking solution (Pierce blocking buffer + 1% SDS) for 45 minutes, then put in contact with a solution of streptavidin conjugated to Raifort peroxidase (1 mg/ml), which was diluted 1:750 in the blocking solution; after 45 minutes of incubation, the membrane was rinsed using a washing solution (PBS with Tween® surfactant, 0.2 % v/v) for 30 minutes. A luminol-based substrate, marketed by Pierce, of black radish peroxidase was sprayed onto the surface of the membrane in order to allow the luminous reaction to occur. The signals were recorded using the Milliflex® Rapid system.

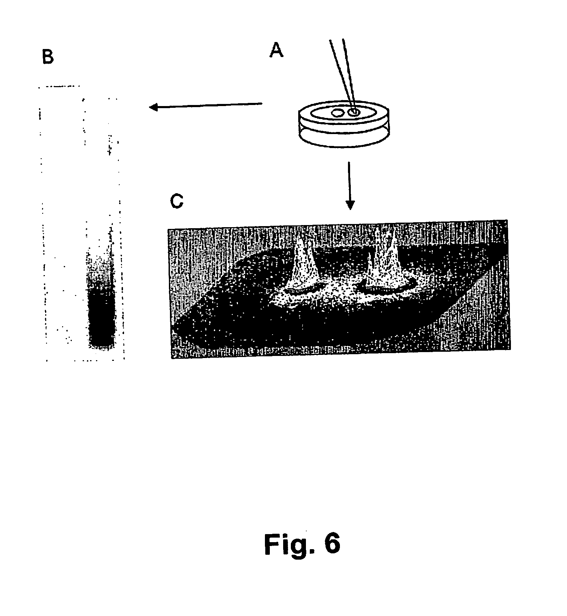

The results are shown in parts B & C of Figure 6. In part C of Figure 6, two amplification peaks corresponding to the initial applications of the cells to the membrane can easily be seen.

In parallel, the solution, having served for amplification, was recovered and analyzed by agarose gel electrophoresis. The result of this is shown in part B of Figure 6. It can easily be seen that part of the amplification products is found in the amplification solution.

This experiment shows that amplification products obtained from a quantity of nucleic acids equivalent to 130 cells of a microorganism such as *Pseudomonas aeruginosa* can be easily detected on membrane. Nevertheless, part of these amplification products is eluted by the membrane and can be detected by electrophoresis.

### Example 3

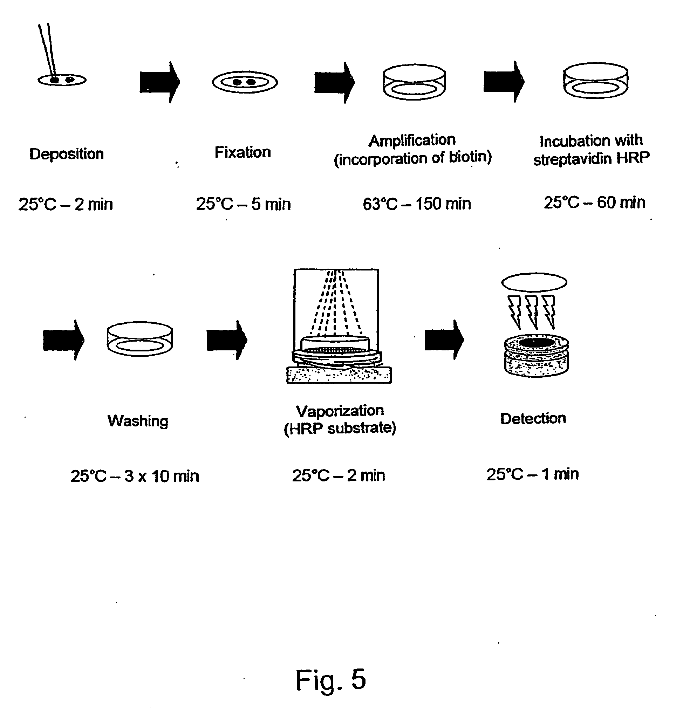

Another test illustrated in Figure 6 can consist of filtering 100 ml of a solution using the device shown in Figure 5.

After filtration, the device is placed on a cellulose pad impregnated with 1.5 ml of the fixation solution (0.25% glutaraldehyde in 95% of denatured alcohol) for 5 minutes at ambient temperature.

The membrane is dried for 5 minutes at ambient temperature, then the lower part of the device is closed using the closure cassette in order to avoid leaks during incubation.

1.5 ml of the amplification solution (2 pmol/µl of FIP & BIP oligonucleotides; 1 pmol/µl of loop F & loop B oligonucleotides; 0.2 pmol/µl of F3 & B3 oligonucleotides in 40 mM Tris HCl pH 8.8, 20 mM KCI, 0.6 M betaine, 1.5 mM each of dNTP + 0.2 mM UTP biotin, 20 mM (NH₄)₂SO₄, 8 mM MgSO₄, 0.1 % Triton® X100 surfactant, 0.005 U/µl AMV RTase, 0.32 U/µl DNA polymerase) is distributed over the membrane of the device then its upper part is closed again with the cover in order to avoid evaporation during incubation at 63°C for 150 minutes.

The membrane is then incubated with a blocking solution for 45 minutes, then put in contact with a streptavidin solution conjugated to Raifort peroxidase (1 mg/ml), which is diluted 1:750 in the blocking solution; After 45 minutes of incubation, the membrane was rinsed using a solution of PBS with Tween® surfactant,0.2 % v/v for 30 minutes. A luminol-based substrate of black radish peroxidase, marketed by Pierce, is sprayed onto the surface of the membrane in order to allow the luminous reaction to occur. The signals are recorded using the Milliflex® Rapid system.

## Claims

1. Method for the specific detection on a membrane of one or more microorganisms present in a liquid, a gas or on a surface, **characterized in that** it comprises the following steps:
i) the liquid, the gas or the surface is put in contact with the membrane, so as to trap the microorganism or microorganisms on the membrane;
ii) said microorganisms are treated on said membrane with a fixation solution containing a cross-linker selected from glutaraldehyde, formaldehyde and/or paraformaldehyde;
iii) nucleic acids present in the microorganism or microorganisms are specifically isothermally amplified on said membrane in order to obtain amplification products;
iv) the obtained amplification products are detected directly on said membrane where the microorganisms have been trapped.

2. Method according to claim 1, **characterized in that** the microorganism or microorganisms, or their nucleic acids, are fixed on a PVDF or polyamide-based membrane.

3. Method according to claim 1 or 2, **characterized in that** said method allows individual counting of the microorganisms trapped on said membrane.

4. Method according to any one of claims 1 to 3, **characterized in that** in step i) the microorganism or microorganisms are trapped by passing a liquid or a gas through the membrane.

5. Method according to any one of claims 1 to 3, **characterized in that** in step a) the microorganism or microorganisms present in a gas or on a surface are trapped by impaction of the microorganisms on the membrane.

6. Method according to any one of claims 1 or 5, **characterized in that** the microorganism or microorganisms to be detected are contained in an aqueous medium.

7. Method according to any one of claims 1 to 6, **characterized in that** the microorganism or microorganisms to be detected are contained in air.

8. Method according to any one of claims 1 to 7, **characterized in that** the nucleic acids amplified in step iii) consist of DNAs.

9. Method according to claim 8, **characterized in that** the nucleic acids amplified in step iii) consist of cDNAs obtained by a specific additional reverse transcription step carried out starting from the RNAs contained in the microorganism or microorganisms.

10. Method according to claim 9, **characterized in that** the RNAs starting from which the reverse transcription step is carried out are the messenger RNAs contained in the microorganism or microorganisms.

11. Method according to any one of claims 8 to 10, **characterized in that** the nucleic acid, or the cDNA obtained by reverse transcription, is amplified in step iii) by a LAMP-type amplification technique.

12. Method according to claim 11, **characterized in that** the LAMP-type amplification technique comprises a phase in which loop F and loop B primers are hybridized at the level of the loops present in the amplification products, in order to increase the speed of formation of said amplification products.

13. Method according to any one of claims 1 to 12, **characterized in that** the amplification products which are detected in step iv) consist of DNAs.

14. Method according to any one of claim 8 to 10, **characterized in that** the nucleic acids, or the cDNA obtained by reverse transcription, are amplified in step iv) by NASBA (nucleic acid sequence based amplification), or TMA (transcription mediated amplification) technique.

15. Method according to any one of claims 1 to 14, **characterized in that** a marker is incorporated into the amplification products obtained in step iii) allowing their detection in step iv).

16. Method according to claim 15, **characterized in that** the marker incorporated into the amplification products is a marked purine or pyrimidine base.

17. Method according to claim 15, **characterized in that** the marker incorporated into the amplification products consists of primers which are marked.

18. Method according to claim 17, **characterized in that** the marked primers, incorporated into the amplification products, consist of loop F and/or loop B primers as defined in claim 12.

19. Method according to any one of the claims 15 to 18, **characterized in that** the marker incorporated into the amplification products is coupled with a ligand which is then reacted with a marked molecule capable of interacting with this ligand.

20. Method according to claim 19, **characterized in that** the ligand is a biotin.

21. Method according to claim 20, **characterized in that** in step iv) the amplification products comprising the biotin are put in contact with the streptavidin coupled to a molecule allowing it to be detected.

22. Method according to claim 19, **characterized in that** the ligand consists of an antigen or an antibody which is then reacted with the marked antibodies or antigens.

23. Method according to claim 22, **characterized in that** the marker incorporated into the amplification products in step iii) is coupled to a dioxygenin.

24. Method according to claim 23, **characterized in that** in step iv) the amplification products comprising the dioxygenin are put in contact with an anti-dioxygenin antibody coupled to a molecule allowing it to be detected.

25. Method according to one of claims 19 and 24, **characterized in that** the marked molecule capable of interacting with the ligand is conjugated with black radish peroxidase or the soya seed peroxidase.

26. Method according to one of claims 19 and 24, **characterized in that** the marked molecule capable of interacting with this ligand is conjugated with alkaline phosphatase.

27. Method according to any one of claims 15 to 26, **characterized in that** the marker incorporated into the amplification products allows detection by fluorescence.

28. Method according to claim 27, **characterized in that** the molecules allowing the detection by fluorescence of the amplification products have an emission spectrum in the near infrared.

29. Method according to any one of claims 1 to 10, **characterized in that** in step iv) the amplification products obtained in step iii) are hybridized with marked specific hybridization probes.

30. Method according to claim 29, in which the hybridization probes are marked using a fluorescent molecule, a ligand, an antibody or an antigen as defined in any one of claims 19 to 29.

31. Method according to one of claims 29 and 30, **characterized in that** the marked specific hybridization probe is of PNA type.

32. Method according to any one of claims 1 to 31, **characterized in that** the membrane used has pores of an average diameter comprised between 0.1 and 1.5 microns, preferably between 0.15 and 0.8 microns, more preferentially between 0.2 and 0.6 microns.

33. Method according to any one of claims 1 to 32, **characterized in that** the membrane is constituted by one or more materials chosen from polytetrafluoroethylene, poly(vinylidene) fluoride, polycarbonate, polyamide, polysulphone, polyethersulfone, acetyl cellulose, mixed cellulosic esters and nitrocellulose.

34. Method according to any one of claims 1 to 33, **characterized in that** it additionally comprises the step of culturing the microorganism or microorganisms trapped on the membrane in step ii) by putting said membrane in contact with a nutritive medium.

35. Method according to any one of claims 1 to 34, **characterized in that** it additionally comprises the step of lysis of the wall of the microorganism or microorganisms trapped on the membrane in order to release the nucleic acids contained in the microorganism or microorganisms.

36. Method according to any one of claims 1 to 35, **characterized in that** it additionally comprises the step of detecting the presence of the living microorganism or microorganisms by ATP bioluminescence.

37. Method according to any one of claims 1 to 36, **characterized in that** several types of microorganisms originating from the same fluid can be detected in parallel in a specific manner on the same membrane.

38. Method according to any one of claims 1 to 37, **characterized in that** the microorganism or microorganisms to be detected are chosen from bacteria, viruses, protozoa, mycoplasmas, molds and yeasts.

39. Method according to any one of claims 1 to 38, **characterized in that** the microorganism or microorganisms to be detected are present in a liquid which is water.

40. Method according to any one of claims 1 to 39, **characterized in that** the microorganism is the *Pseudomonas aeruginosa* bacteria.

41. Method for the specific detection on a membrane of one or more bacteria of the species *Pseudomonas aeruginosa* contained in a liquid, **characterized in that** it comprises the following steps:
i) the liquid is passed through a membrane;
ii) the bacteria contained in the liquid are trapped on the membrane;
iii) said bacteria are treated on said membrane with a fixation solution containing a cross-linker selected from glutaraldehyde, formaldehyde and/or paraformaldehyde;
iv) nucleic acids of *Pseudomonas aeruginosa* contained in these bacteria are specifically isothermally amplified on said membrane with the help of a LAMP-type amplification by using at least one primer comprising a sequence taken from SEQ ID No.2, SEQ ID No.3, SEQ ID No.4, SEQ ID No.5, SEQ ID No.6, and SEQ ID No.7;
v) the amplification products obtained on the membrane are detected.

42. Method according to claim 41, **characterized in that** a reverse transcription step on the RNAs contained in the bacteria in order to obtain a cDNA comprising the sequence SEQ ID No.1, the LAMP-type amplification being carried out starting from said cDNA.

43. Method according to any one of claims 1 to 42, **characterized in that** the membrane is a PVDF membrane.

44. A kit for the specific detection on a membrane of one or more microorganisms comprising:
- a PVDF membrane ;
- a fixing solution containing glutaraldehyde, formaldehyde or paraformaldehyde; and
- at least one specific primer allowing isothermal amplification of the nucleic acids from the microorganisms.

45. A kit for the specific detection on a membrane of one or more microorganisms according to claim 44, comprising:
- a PVDF membrane ;
- a fixing solution containing glutaraldehyde, formaldehyde or paraformaldehyde; and
- at least one specific FIP primer allowing isothermal amplification of the nucleic acids from the microorganisms by a LAMP type amplification technique.

46. A kit according to claim 44, for the specific detection on a membrane of the *Pseudomonas aeruginosa* bacterium comprising:
- a PVDF membrane;
- a fixing solution containing glutaraldehyde, formaldehyde or paraformaldehyde; and
- at least one oligonucleotide for the specific isothermal amplification of *Pseudomonas aeruginosa* nucleic acids comprising a nucleotide sequence chosen from the sequences SEQ ID Nos.2 to 7.

47. Device for the amplification of nucleic acids suitable for implementing the method according to claims 1 to 41, comprising a filter (1) for receiving the nucleic acids to be amplified, this device being **characterized in that** it contains a body (2) delimiting an internal volume, a flat impermeable wall (3) and a grid (4) forming a plurality of cells on the surface of the filter (1), the body (2) being provided with means for keeping the filter (1) in contact, in this internal volume, with said flat impermeable wall (3).

48. Device according to claim 47, **characterized in that** said cells make it possible to contain a volume comprised between 1 and 100 µl, preferably between 3 and 10 µl, of a reaction solution.

49. Device according to one of claims 47 and 48, **characterized in that** the body (2) has a shoulder (5) suitable for cooperating with the periphery of the filter (1).

50. Device according to claim 49, **characterized in that** the filter (1) is fixed by its periphery against said shoulder (5).

51. Device according to any one of claims 49 to 50, **characterized in that** the flat wall (3) secures the periphery of the filter (1) against said shoulder (5).

52. Device according to any one of claims 47 to 51, **characterized in that** the grid (4) is made of a single piece with the body (2).

53. Device according to any one of claims 47 to 52, **characterized in that** the flat wall (3) is detachable vis-à-vis the body (2).

54. Device according to claim 53, **characterized in that** the flat wall (3) has a skirt (6) extending transversely and **in that** the body (2) has a sleeve (7) intended to cooperate with the skirt (6).

55. Device according to any one of claims 47 to 54, **characterized in that** it also has a detachable cover (8) which is suitable for being arranged opposite the grid (4) to close said internal volume.

56. Device according to any one of claims 47 to 55, **characterized in that** it has a reservoir (9) attached to the body (2).

57. Device according to claim 56, **characterized in that** the reservoir (9) is attached to the body (2) by a frangible zone (10).

58. Device according to claim 56, **characterized in that** it can also have a detachable cover suitable for alternately closing the reservoir (9) and said internal volume at the level of the frangible zone (10).

59. Device according to any one of claims 47 to 58, **characterized in that** the flat wall (3) has a drainage orifice (11).

60. Device according to any one of claims 47 to 59, **characterized in that** the filter (1) is a membrane.

## Patentansprüche

1. Verfahren zur spezifischen Detektion von einem oder mehreren Mikroorganismen, die in einer Flüssigkeit, einem Gas oder auf einer Oberfläche vorhanden sind, auf einer Membran, **dadurch gekennzeichnet, dass** es die folgenden Stufen umfasst:
i) die Flüssigkeit, das Gas oder die Oberfläche werden mit der Membran so in Kontakt gebracht, dass der Mikroorganismus bzw. die Mikroorganismen auf der Membran aufgefangen werden;
ii) die Mikroorganismen werden auf der Membran mit einer Fixierlösung behandelt, die ein Vernetzungsmittel enthält, das aus Glutaraldehyd, Formaldehyd und/oder Paraformaldehyd ausgewählt ist;
iii) Nucleinsäuren, die in dem Mikroorganismus oder den Mikroorganismen vorhanden sind, werden auf der Membran spezifisch isotherm amplifiziert, um Amplifikationsprodukte zu erhalten;
iv) die erhaltenen Amplifikationsprodukte werden direkt auf der Membran, auf der die Mikroorganismen aufgefangen wurden, detektiert.

2. Verfahren nach Anspruch 1, **dadurch gekennzeichnet, dass** der Mikroorganismus bzw. die Mikroorganismen oder deren Nucleinsäuren auf einer Membran auf PVDF- oder Polyamidbasis fixiert werden.

3. Verfahren nach Anspruch 1 oder 2, **dadurch gekennzeichnet, dass** das Verfahren ein individuelles Zählen der auf der Membran eingefangenen Mikroorganismen ermöglicht.

4. Verfahren nach einem der Ansprüche 1 bis 3, **dadurch gekennzeichnet, dass** in Stufe i) der Mikroorganismus bzw. die Mikroorganismen durch Hindurchleiten einer Flüssigkeit oder eines Gases durch die Membran eingefangen werden.

5. Verfahren nach einem der Ansprüche 1 bis 3, **dadurch gekennzeichnet, dass** in Stufe a) der Mikroorganismus bzw. die Mikroorganismen, die in einem Gas oder auf einer Oberfläche vorhanden sind, durch Aufprallen der Mikroorganismen auf die Membran aufgefangen werden.

6. Verfahren nach einem der Ansprüche 1 oder 5, **dadurch gekennzeichnet, dass** der Mikroorganismus bzw. die Mikroorganismen, die zu detektieren sind, in einem wässrigen Medium enthalten sind.

7. Verfahren nach einem der Ansprüche 1 bis 6, **dadurch gekennzeichnet, dass** der Mikroorganismus bzw. die Mikroorganismen, die zu detektieren sind, in Luft enthalten sind.

8. Verfahren nach einem der Ansprüche 1 bis 7, **dadurch gekennzeichnet, dass** die in Stufe iii) amplifizierten Nucleinsäuren aus DNAs bestehen.

9. Verfahren nach Anspruch 8, **dadurch gekennzeichnet, dass** die in Stufe iii) amplifizierten Nucleinsäuren aus cDNAs bestehen, die durch eine spezifische zusätzliche Reverse-Transkription-Stufe, die ausgehend von den in dem Mikroorganismus bzw. den Mikroorganismen enthaltenen RNAs durchgeführt wurde, erhalten werden.

10. Verfahren nach Anspruch 9, **dadurch gekennzeichnet, dass** die RNAs, von denen ausgehend die Reverse-Transkription-Stufe durchgeführt wird, die messenger-RNAs, die in dem Mikroorganismus bzw. den Mikroorganismen enthalten sind, sind.

11. Verfahren nach einem der Ansprüche 8 bis 10, **dadurch gekennzeichnet, dass** die Nucleinsäure oder die durch reverse Transkription erhaltene cDNA in Stufe iii) durch eine LAMP-Amplifikationstechnik amplifiziert wird.

12. Verfahren nach Anspruch 11, **dadurch gekennzeichnet, dass** die LAMP-Amplifikationstechnik eine Phase umfasst, in der eine Hybridisierung von Loop-F- und Loop-B-Primern auf der Ebene der in den Amplifikationsprodukten vorhandenen Loops erfolgt, um die Geschwindigkeit der Bildung der Amplifikationsprodukte zu erhöhen.

13. Verfahren nach einem der Ansprüche 1 bis 12, **dadurch gekennzeichnet, dass** die Amplifikationsprodukte, die in Stufe iv) detektiert werden, aus DNAs bestehen.

14. Verfahren nach einem der Ansprüche 8 bis 10, **dadurch gekennzeichnet, dass** die Nucleinsäuren oder die durch reverse Transkription erhaltene cDNA in Stufe iv) durch die NASBA (Nucleic Acid Sequence Based Amplification)- oder TMA (Transcription Mediated Amplifikation)-Technik amplifiziert werden.

15. Verfahren nach einem der Ansprüche 1 bis 14, **dadurch gekennzeichnet, dass** ein Marker in die in Stufe iii) erhaltenen Amplifikationsprodukte eingebaut wird, was deren Detektion in Stufe iv) ermöglicht.

16. Verfahren nach Anspruch 15, **dadurch gekennzeichnet, dass** der in die Amplifikationsprodukte eingebaute Marker eine markierte Purin- oder Pyrimidinbase ist.

17. Verfahren nach Anspruch 15, **dadurch gekennzeichnet, dass** der in die Amplifikationsprodukte eingebaute Marker aus Primern besteht, die markiert sind.

18. Verfahren nach Anspruch 17, **dadurch gekennzeichnet, dass** die markierten Primer, die in die Amplifikationsprodukte eingebaut sind, aus Loop-F- und/oder Loop-B-Primern gemäß der Definition in Anspruch 12 bestehen.

19. Verfahren nach einem der Ansprüche 15 bis 18, **dadurch gekennzeichnet, dass** der in die Amplifikationsprodukte eingebaute Marker mit einem Liganden gekoppelt wird, der dann mit einem markierten Molekül, das mit diesem Liganden wechselwirken kann, reagieren gelassen wird.

20. Verfahren nach Anspruch 19, **dadurch gekennzeichnet, dass** der Ligand ein Biotin ist.

21. Verfahren nach Anspruch 20, **dadurch gekennzeichnet, dass** in Stufe iv) die das Biotin umfassenden Amplifikationsprodukte mit dem an ein Molekül gekoppelten Streptavidin in Kontakt gebracht werden, wodurch eine Detektion des Moleküls ermöglicht wird.

22. Verfahren nach Anspruch 19, **dadurch gekennzeichnet, dass** der Ligand aus einem Antigen oder einem Antikörper besteht, die dann mit den markierten Antikörpern bzw. Antigenen reagieren gelassen werden.

23. Verfahren nach Anspruch 22, **dadurch gekennzeichnet, dass** der in die Amplifikationsprodukte in Stufe iii) eingebaute Marker an ein Dioxygenin gekoppelt wird.

24. Verfahren nach Anspruch 23, **dadurch gekennzeichnet, dass** in Stufe iv) die das Dioxygenin umfassenden Amplifikationsprodukte mit einem an ein Molekül gekoppelten Anti-Dioxygenin-Antikörper in Kontakt gebracht werden, wodurch eine Detektion des Moleküls ermöglicht wird.

25. Verfahren nach einem der Ansprüche 19 und 24, **dadurch gekennzeichnet, dass** das markierte Molekül, das zur Wechselwirkung mit dem Liganden fähig ist, mit Schwarzer-Rettich-Peroxidase oder Sojabohnen-Peroxidase konjugiert ist.

26. Verfahren nach einem der Ansprüche 19 und 24, **dadurch gekennzeichnet, dass** das markierte Molekül, das zur Wechselwirkung mit diesem Liganden fähig ist, mit alkalischer Phosphatase konjugiert ist.

27. Verfahren nach einem der Ansprüche 15 bis 26, **dadurch gekennzeichnet, dass** der in die Amplifikationsprodukte eingebaute Marker eine Detektion durch Fluoreszenz ermöglicht.

28. Verfahren nach Anspruch 27, **dadurch gekennzeichnet, dass** die Moleküle, die die Detektion der Amplifikationsprodukte durch Fluoreszenz ermöglichen, ein Emissionsspektrum im nahen Infrarot aufweisen.

29. Verfahren nach einem der Ansprüche 1 bis 10, **dadurch gekennzeichnet, dass** in Stufe iv) die in Stufe iii) erhaltenen Amplifikationsprodukte mit markierten spezifischen Hybridisierungssonden hybridisiert werden.

30. Verfahren nach Anspruch 29, wobei die Hybridisierungssonden unter Verwendung eines fluoreszierenden Moleküls, eines Liganden, eines Antikörpers oder eines Antigens gemäß der Definition in einem der Ansprüche 19 bis 29 markiert sind.

31. Verfahren nach einem der Ansprüche 29 und 30, **dadurch gekennzeichnet, dass** die markierte spezifische Hybridisierungssonde vom PNA-Typ ist.

32. Verfahren nach einem der Ansprüche 1 bis 31, **dadurch gekennzeichnet, dass** die verwendete Membran Poren mit einem mittleren Durchmesser, der zwischen 0,1 und 1,5 µm, vorzugsweise zwischen 0,15 und 0,8 µm, noch günstiger zwischen 0,2 und 0,6 µm liegt, aufweist.

33. Verfahren nach einem der Ansprüche 1 bis 32, **dadurch gekennzeichnet, dass** die Membran aus einem oder mehreren Materialien besteht, die aus Polytetrafluorethylen, Poly(vinyliden)fluorid, Polycarbonat, Polyamid, Polysulfon, Polyethersulfon, Acetylcellulose, Celluloseestergemischen und Nitrocellulose ausgewählt sind.

34. Verfahren nach einem der Ansprüche 1 bis 33, **dadurch gekennzeichnet, dass** es zusätzlich die Stufe des Kultivierens des Mikroorganismus bzw. der Mikroorganismen, die auf der Membran in Stufe ii) eingefangen wurden, durch Inkontaktbringen der Membran mit einem Nährmedium umfasst.

35. Verfahren nach einem der Ansprüche 1 bis 34, **dadurch gekennzeichnet, dass** es zusätzlich die Stufe einer Lyse der Wand des Mikroorganismus bzw. der Mikroorganismen, die auf der Membran eingefangen sind, um die in dem Mikroorganismus bzw. den Mikroorganismen enthaltenen Nucleinsäuren freizusetzen, umfasst.

36. Verfahren nach einem der Ansprüche 1 bis 35, **dadurch gekennzeichnet, dass** es zusätzlich die Stufe der Detektion des Vorhandenseins des lebenden Mikroorganismus bzw. der lebenden Mikroorganismen durch ATP-Biolumineszenz umfasst.

37. Verfahren nach einem der Ansprüche 1 bis 36, **dadurch gekennzeichnet, dass** mehrere Arten von Mikroorganismen, die von dem gleichen Fluidum stammen, parallel auf spezifische Weise auf der gleichen Membran detektiert werden können.

38. Verfahren nach einem der Ansprüche 1 bis 37, **dadurch gekennzeichnet, dass** der Mikroorganismus bzw. die Mikroorganismen, die zu detektieren sind, aus Bakterien, Viren, Protozoen, Mycoplasmen, Schimmelpilzen und Hefen ausgewählt sind.

39. Verfahren nach einem der Ansprüche 1 bis 38, **dadurch gekennzeichnet, dass** der Mikroorganismus bzw. die Mikroorganismen, die zu detektieren sind, in einer Flüssigkeit vorhanden sind, wobei diese Wasser ist.

40. Verfahren nach einem der Ansprüche 1 bis 39, **dadurch gekennzeichnet, dass** der Mikroorganismus das Bakterium *Pseudomonas aeruginosa* ist.

41. Verfahren zur spezifischen Detektion von einem oder mehreren Bakterien der Art *Pseudomonas aeruginosa,* die in einer Flüssigkeit enthalten sind, auf einer Membran, **dadurch gekennzeichnet, dass** das Verfahren die folgenden Stufen umfasst:
i) die Flüssigkeit wird durch eine Membran hindurch geleitet;
ii) die in der Flüssigkeit enthaltenen Bakterien werden auf der Membran aufgefangen;
iii) die genannten Bakterien werden auf der Membran mit einer Fixierungslösung behandelt, die ein Vernetzungsmittel enthält, das aus Glutaraldehyd, Formaldehyd und/oder Paraformaldehyd ausgewählt ist;
iv) Nucleinsäuren von *Pseudomonas aeruginosa,* die in diesen Bakterien enthalten sind, werden auf der Membran mit Hilfe einer LAMP-Amplifikation unter Verwendung von mindestens einem Primer, der eine Sequenz umfasst, die aus SEQ ID NO. 2, SEQ ID NO. 3, SEQ ID NO. 4, SEQ ID NO. 5, SEQ ID NO. 6 und SEQ ID NO. 7 ausgewählt ist, spezifisch isotherm amplifiziert;
v) die auf der Membran erhaltenen Amplifikationsprodukte werden detektiert.

42. Verfahren nach Anspruch 41, **dadurch gekennzeichnet, dass** eine Reverse-Transkription-Stufe an den in den Bakterien enthaltenen RNAs durchgeführt wird, um eine die Sequenz SEQ ID NO. 1 umfassende cDNA zu erhalten, wobei die LAMP-Amplifikation ausgehend von der cDNA durchgeführt wird.

43. Verfahren nach einem der Ansprüche 1 bis 42, **dadurch gekennzeichnet, dass** die Membran eine PVDF-Membran ist.

44. Kit zur spezifischen Detektion von einem oder mehreren Mikroorganismen auf einer Membran, umfassend:
- eine PVDF-Membran;
- eine Fixierlösung, die Glutaraldehyd, Formaldehyd oder Paraformaldehyd enthält; und
- mindestens einen spezifischen Primer, der eine isotherme Amplifikation der Nucleinsäuren von den Mikroorganismen ermöglicht.

45. Kit zur spezifischen Detektion von einem oder mehreren Mikroorganismen auf einer Membran nach Anspruch 44, umfassend:
- eine PVDF-Membran;
- eine Fixierlösung, die Glutaraldehyd, Formaldehyd oder Paraformaldehyd enthält; und
- mindestens einen spezifischen FIP-Primer, der eine isotherme Amplifikation der Nucleinsäuren von den Mikroorganismen durch eine LAMP-Amplifikationstechnik ermöglicht.

46. Kit nach Anspruch 44, zur spezifischen Detektion des Bakterium *Pseudomonas aeruginosa* auf einer Membran, umfassend:
- eine PVDF-Membran;
- eine Fixierlösung, die Glutaraldehyd, Formaldehyd oder Paraformaldehyd enthält; und
- mindestens ein Oligonucleotid zur spezifischen isothermen Amplifikation von *Pseudomonas aeruginosa*-Nucleinsäuren, das eine Nucleotidsequenz umfasst, die aus den Sequenzen SEQ ID NO. 2 bis 7 ausgewählt ist.

47. Vorrichtung zur Amplifikation von Nucleinsäuren, die zur Implementierung des Verfahrens gemäß den Ansprüchen 1 bis 41 geeignet ist, umfassend ein Filter (1) zur Aufnahme der zu amplifizierenden Nucleinsäuren, wobei diese Vorrichtung **dadurch gekennzeichnet ist, dass** sie einen Körper (2), der ein Innenvolumen begrenzt, eine plane undurchlässige Wand (3) und ein Gitter (4), das auf der Oberfläche des Filters (1) eine Mehrzahl von Zellen bildet, enthält, wobei der Körper (2) mit Mitteln ausgestattet ist, um das Filter (1) in diesem Innenvolumen mit der planen undurchlässigen Wand (3) in Kontakt zu halten.

48. Vorrichtung nach Anspruch 47, **dadurch gekennzeichnet, dass** es die Zellen möglich machen, dass sie ein Volumen zwischen 1 und 100 µl, vorzugsweise zwischen 3 und 10 µl, einer Reaktionslösung enthalten.

49. Vorrichtung nach einem der Ansprüche 47 und 48, **dadurch gekennzeichnet, dass** der Körper (2) eine Schulter (5) aufweist, die zur Kooperation mit dem Umfang des Filters (1) geeignet ist.

50. Vorrichtung nach Anspruch 49, **dadurch gekennzeichnet, dass** das Filter (1) durch dessen Umfang an der Schulter (5) befestigt ist.

51. Vorrichtung nach einem der Ansprüche 49 bis 50, **dadurch gekennzeichnet, dass** die plane Wand (3) den Umfang des Filters (1) an der Schulter (5) sicher befestigt.

52. Vorrichtung nach einem der Ansprüche 47 bis 51, **dadurch gekennzeichnet, dass** das Gitter (4) einstückig mit dem Körper (2) ausgebildet ist.

53. Vorrichtung nach einem der Ansprüche 47 bis 52, **dadurch gekennzeichnet, dass** die plane Wand (3) vom Körper (2) abtrennbar ist.

54. Vorrichtung nach Anspruch 53, **dadurch gekennzeichnet, dass** die plane Wand (3) einen quer abstehenden Kragen (6) aufweist und dass der Körper (2) eine Hülse (7) aufweist, die mit dem Kragen (6) kooperiert.

55. Vorrichtung nach einem der Ansprüche 47 bis 54, **dadurch gekennzeichnet, dass** sie auch eine abnehmbare Abdeckung (8) aufweist, die für eine Anbringung entgegengesetzt zum Gitter (4) zum Schließen des Innenvolumens geeignet ist.

56. Vorrichtung nach einem der Ansprüche 47 bis 55, **dadurch gekennzeichnet, dass** sie einen an dem Körper (2) angebrachten Vorratsbehälter (9) aufweist.

57. Vorrichtung nach Anspruch 56, **dadurch gekennzeichnet, dass** der Vorratsbehälter (9) an dem Körper (2) durch eine brechbare Zone (10) angebracht ist.

58. Vorrichtung nach Anspruch 56, **dadurch gekennzeichnet, dass** sie auch eine abnehmbare Abdeckung aufweisen kann, die zum alternativen Verschließen des Vorratsbehälters (9) und des Innenvolumens auf der Höhe der brechbaren Zone (10) geeignet ist.

59. Vorrichtung nach einem der Ansprüche 47 bis 58, **dadurch gekennzeichnet, dass** die plane Wand (3) eine Drainageöffnung (11) aufweist.

60. Vorrichtung nach einem der Ansprüche 47 bis 59, **dadurch gekennzeichnet, dass** das Filter (1) eine Membran ist.

## Revendications

1. Procédé de détection spécifique sur une membrane d'un ou plusieurs microorganismes présents dans un liquide, un gaz ou sur une surface, **caractérisé en ce qu'**il comprend les étapes suivantes :
i) le liquide, le gaz ou la surface est mis en contact avec la membrane, de manière à capter le ou les microorganismes sur la membrane ;
ii) lesdits microorganismes sont traités sur ladite membrane avec une solution de fixation contenant un agent de réticulation choisi entre le glutaraldéhyde, le formaldéhyde et/ou le paraformaldéhyde ;
iii) les acides nucléiques présents dans le ou les microorganismes sont amplifiés spécifiquement de manière isotherme sur ladite membrane afin d'obtenir des produits d'amplification ;
iv) les produits d'amplification obtenus sont détectés directement sur ladite membrane où les microorganismes ont été captés.

2. Procédé selon la revendication 1, **caractérisé en ce que** le ou les microorganismes, ou leurs acides nucléiques, sont fixés sur une membrane à base de PVDF ou de polyamide.

3. Procédé selon la revendication 1 ou 2, **caractérisé en ce qu'**il permet d'énumérer individuellement des microorganismes captés sur ladite membrane.

4. Procédé selon l'une quelconque des revendications 1 à 3, **caractérisé en ce que**, dans l'étape i), le ou les microorganismes sont captés par passage d'un liquide ou d'un gaz à travers la membrane.

5. Procédé selon l'une quelconque des revendications 1 à 3, **caractérisé en ce que**, dans l'étape a), le ou les microorganismes présents dans un gaz ou sur une surface sont captés par impact des microorganismes sur la membrane.

6. Procédé selon l'une quelconque des revendications 1 ou 5, **caractérisé en ce que** le ou les microorganismes à détecter sont présents dans un milieu aqueux.

7. Procédé selon l'une quelconque des revendications 1 à 6, **caractérisé en ce que** le ou les microorganismes à détecter sont présents dans l'air.

8. Procédé selon l'une quelconque des revendications 1 à 7, **caractérisé en ce que** les acides nucléiques amplifiés dans l'étape iii) consistent en des ADN.

9. Procédé selon la revendication 8, **caractérisé en ce que** les acides nucléiques amplifiés dans l'étape iii) consistent en des ADNc obtenus par une étape de transcription inverse additionnelle spécifique mise en oeuvre en partant des ARN présents dans le ou les microorganismes.

10. Procédé selon la revendication 9, **caractérisé en ce que** les ARN de départ à partir desquels l'étape de transcription inverse est effectuée sans les ARN messagers présents dans le ou les microorganismes.

11. Procédé selon l'une quelconque des revendications 8 à 10, **caractérisé en ce que** l'acide nucléique, ou l'ADNc obtenu par transcription inverse, est amplifié dans l'étape iii) par une technique d'amplification du type LAMP.

12. Procédé selon la revendication 11, **caractérisé en ce que** la technique d'amplification du type LAMP comprend une phase dans laquelle les amorces de loop F et de loop B sont hybridées au niveau des boucles présentes dans les produits d'amplification, afin d'augmenter la vitesse de formation desdits produits d'amplification.

13. Procédé selon l'une quelconque des revendications 1 à 12, **caractérisé en ce que** les produits d'amplification qui sont détectés dans l'étape iv) consistent en des ADN.

14. Procédé selon l'une quelconque des revendications 8 à 10, **caractérisé en ce que** les acides nucléiques, ou les ADNc obtenus par transcription inverse sont amplifiés dans l'étape iv) par la technique NASBA (amplification basée sur une séquence d'acide nucléique) ou la technique TMA (amplification à médiation par transcription).

15. Procédé selon l'une quelconque des revendications 1 à 14, **caractérisé en ce qu'**un marqueur est incorporé aux produits d'amplification obtenus dans l'étape iii), ce qui permet leur détection dans l'étape iv).

16. Procédé selon la revendication 15, **caractérisé en ce que** le marqueur incorporé aux produits d'amplification est une base purique ou pyrimidique marquée.

17. Procédé selon la revendication 15, **caractérisé en ce que** le marqueur incorporé aux produits d'amplification consiste en amorces qui sont marquées.

18. Procédé selon la revendication 17, **caractérisé en ce que** les amorces marquées, incorporées aux produits d'amplification, consistent en amorces de boucle F et/ou de boucle B telles que définies dans la revendication 12.

19. Procédé selon l'une quelconque des revendications 15 à 18, **caractérisé en ce que** le marqueur incorporé aux produits d'amplification est couplé avec un ligand que l'on fait réagir avec une molécule marquée capable d'interagir avec ce ligand.

20. Procédé selon la revendication 19, **caractérisé en ce que** le ligand est une biotine.

21. Procédé selon la revendication 20, **caractérisé en ce que**, dans l'étape iv), les produits d'amplification comprenant la biotine sont mis en contact avec la streptavidine couplée à une molécule, ce qui permet sa détection.

22. Procédé selon la revendication 19, **caractérisé en ce que** le ligand consiste en un antigène ou un anticorps que l'on fait réagir avec les anticorps ou antigènes marqués.

23. Procédé selon la revendication 22, **caractérisé en ce que** le marqueur incorporé aux produits d'amplification dans l'étape iii) est couplé avec une dioxygénine.

24. Procédé selon la revendication 23, **caractérisé en ce que**, dans l'étape iv), les produits d'amplification comprenant la dioxygénine sont mis en contact avec un anticorps anti-dioxygénine couplé à une molécule, ce qui permet sa détection.

25. Procédé selon l'une des revendications 19 et 24, **caractérisé en ce que** la molécule marquée capable d'interagir avec le ligand est conjuguée avec la péroxydase de radis noir ou la péroxydase de graines de soja.

26. Procédé selon l'une des revendications 19 et 24, **caractérisé en ce que** la molécule marquée capable d'interagir avec ce ligand est conjuguée avec de la phosphatase alcaline.

27. Procédé selon l'une quelconque des revendications 15 à 26, **caractérisé en ce que** le marqueur incorporé aux produits d'amplification permet une détection par fluorescence.

28. Procédé selon la revendication 27, **caractérisé en ce que** les molécules permettant la détection par fluorescence des produits d'amplification ont un spectre d'émission dans l'infrarouge proche.

29. Procédé selon l'une quelconque des revendications 1 à 10, **caractérisé en ce que**, dans l'étape iv), les produits d'amplification obtenus dans l'étape iii) sont hybridés avec des sondes d'hybridation spécifiques marquées.

30. Procédé selon la revendication 29, dans lequel les sondes d'hybridation sont marquées en utilisant une molécule fluorescente, un ligand, un anticorps ou un antigène tels que définis dans l'une quelconque des revendications 19 à 29.

31. Procédé selon l'une des revendications 29 et 30, **caractérisé en ce que** la sonde d'hybridation spécifique marquée est du type PNA.

32. Procédé selon l'une quelconque des revendications 1 à 31, **caractérisé en ce que** la membrane utilisée a des pores ayant un diamètre moyen compris entre 0,1 et 1,5 micromètre, avantageusement entre 0,15 et 0,8 micromètre, plus avantageusement entre 0,2 et 0,6 micromètre.

33. Procédé selon l'une quelconque des revendications 1 à 32, **caractérisé en ce que** la membrane est constituée d'une ou plusieurs matières choisies parmi le polytétrafluoréthylène, le fluorure de poly(vinylidène), le polycarbonate, le polyamide, le polysulfone, le polyéthersulfone, l'acétylcellulose, des esters cellulosiques mixes et la nitrocellulose.

34. Procédé selon l'une quelconque des revendications 1 à 33, **caractérisé en ce qu'**il comprend en outre l'étape de culture du ou des microorganismes captés sur la membrane dans l'étape ii) en mettant ladite membrane en contact avec un milieu nutritif.

35. Procédé selon l'une quelconque des revendications 1 à 34, **caractérisé en ce qu'**il comprend en outre l'étape de lyse de la paroi du ou des microorganismes captés sur la membrane afin de libérer les acides nucléiques présents dans le ou les microorganismes.

36. Procédé selon l'une quelconque des revendications 1 à 35, **caractérisé en ce qu'**il comprend en outre l'étape de détection de la présence du ou des microorganismes vivants par ATP-bioluminescence.

37. Procédé selon l'une quelconque des revendications 1 à 36, **caractérisé en ce que** plusieurs types de microorganismes issus du même fluide peuvent être détectés en parallèle d'une manière spécifique sur la même membrane.

38. Procédé selon l'une quelconque des revendications 1 à 37, **caractérisé en ce que** le ou les microorganismes à détecter sont choisis parmi des bactéries, virus, protozoaires, mycoplasmes, moisissures et levures.

39. Procédé selon l'une quelconque des revendications 1 à 38, **caractérisé en ce que** le ou les microorganismes à détecter sont présents dans un liquide qui est l'eau.

40. Procédé selon l'une quelconque des revendications 1 à 39, **caractérisé en ce que** le microorganisme est la bactérie *Pseudomonas aeruginosa*.

41. Procédé pour la détection spécifique sur une membrane d'une ou plusieurs bactéries de l'espèce *Pseudomonas aeruginosa* présentes dans un liquide, **caractérisé en ce qu'**il comprend les étapes suivantes :
i) le liquide est passé à travers une membrane ;
ii) les bactéries présentes dans le liquide sont captées sur la membrane ;
iii) lesdites bactéries sont traitées sur ladite membrane avec une solution de fixation contenant un agent de réticulation choisi parmi le glutaraldéhyde, le formaldéhyde et/ou le paraformaldéhyde ;
iv) les acides nucléiques de *Pseudomonas aeruginosa* présents dans ces bactéries sont amplifiés spécifiquement de manière isotherme sur ladite membrane au moyen d'une amplification du type LAMP en utilisant au moins une amorce comprenant une séquence choisie parmi les SEQ ID No.2, SEQ ID No.3, SEQ ID No.4, SEQ ID No.5, SEQ ID No.6 et SEQ ID No.7 ;
v) les produits d'amplification obtenus sur la membrane sont détectés.

42. Procédé selon la revendication 41, **caractérisé en ce qu'**une étape de transcription inverse sur les ARN présents dans les bactéries afin d'obtenir un ADNc comprenant la SEQ ID No.1, l'amplification du type LAMP étant effectuée en partant dudit ADNc.

43. Procédé selon l'une quelconque des revendications 1 à 42, **caractérisé en ce que** la membrane est une membrane en PVDF.

44. Kit pour la détection spécifique sur une membrane d'un ou plusieurs microorganismes, comprenant :
- une membrane en PVDF ;
- une solution de fixation contenant du glutaraldéhyde, du formaldéhyde ou du paraformaldéhyde ; et
- au moins une amorce spécifique permettant l'amplification isotherme des acides nucléiques des microorganismes.

45. Kit pour la détection spécifique sur une membrane d'un ou plusieurs microorganismes selon la revendication 44, comprenant :
- une membrane en PVDF ;
- une solution de fixation contenant du glutaraldéhyde, du formaldéhyde ou du paraformaldéhyde ; et
- au moins une amorce FIP spécifique permettant l'amplification isotherme des acides nucléiques des microorganismes par une technique d'amplification du type LAMP.

46. Kit selon la revendication 44, pour la détection spécifique sur une membrane de la bactérie *Pseudomonas aeruginosa*, comprenant :
- une membrane en PVDF ;
- une solution de fixation contenant du glutaraldéhyde, du formaldéhyde ou du paraformaldéhyde ; et
- au moins un oligonucléotide pour l'amplification isotherme spécifique des acides nucléiques de *Pseudomonas aeruginosa* comprenant une séquence de nucléotides choisie parmi les SEQ ID Nos. 2 à 7.

47. Dispositif pour l'amplification d'acides nucléiques, apte à la mise en oeuvre du procédé selon les revendications 1 à 41, comprenant un filtre (1) pour recevoir les acides nucléiques à amplifier, ce dispositif étant **caractérisé en ce qu'**il contient un corps (2) délimitant un volume interne, une paroi imperméable plate (3), une grille (4) formée d'une pluralité d'alvéoles sur la surface du filtre (1), le corps (2) étant muni d'un moyen pour maintenir le filtre (1) en contact, dans ce volume intérieur, avec ladite paroi imperméable plate (3).

48. Dispositif selon la revendication 47, **caractérisé en ce que** lesdites cellules permettent de contenir un volume compris entre 1 et 100 µl, de préférence entre 3 et 10 µl, d'une solution de réaction.

49. Dispositif selon l'une des revendications 47 et 48, **caractérisé en ce que** le corps (2) comporte un épaulement (5) apte à coopérer avec la périphérie du filtre (1).

50. Dispositif selon la revendication 49, **caractérisé en ce que** le filtre (1) est fixé par sa périphérie contre ledit épaulement (5).

51. Dispositif selon l'une quelconque des revendications 49 et 50, **caractérisé en ce que** la paroi plate (3) serre la périphérie du filtre (1) contre ledit épaulement (5).

52. Dispositif selon l'une quelconque des revendications 47 à 51, **caractérisé en ce que** la grille (4) est faite d'une seule pièce avec le corps (2).

53. Dispositif selon l'une quelconque des revendications 47 à 52, **caractérisé en ce que** la paroi plate (3) est amovible par rapport au corps (2).

54. Dispositif selon la revendication 53, **caractérisé en ce que** la paroi plate (3) comporte une jupe (6) s'étendant transversalement et **en ce que** le corps (2) comporte un manchon (7) destiné à coopérer avec la jupe (6).

55. Dispositif selon l'une quelconque des revendications 47 à 54, **caractérisé en ce qu'**il comporte également un couvercle amovible (8) qui est apte à être disposé en vis-à-vis de la grille (4) pour fermer ledit volume intérieur.

56. Dispositif selon l'une quelconque des revendications 47 à 55, **caractérisé en ce qu'**il comporte un réservoir (9) rattaché au corps (2).

57. Dispositif selon la revendication 56, **caractérisé en ce que** le réservoir (9) est rattaché au corps (2) par une zone frangible (10).

58. Dispositif selon la revendication 56, **caractérisé en ce qu'**il peut comporter également un couvercle amovible adapté à fermer alternativement le réservoir (9) et ledit volume interne au niveau de la zone frangible (10).

59. Dispositif selon l'une quelconque des revendications 47 à 58, **caractérisé en ce que** la paroi plate (3) comporte un orifice de drainage (11).

60. Dispositif selon l'une quelconque des revendications 47 à 59, **caractérisé en ce que** le filtre (1) est une membrane.


### SEQUENCE LISTING

<110> MILLIPORE CORPORATION
<120> Method for the detection and characterization of micoorganisms on a
   filter
<130> BIF 116630 WO
<150> FR 0504379
   <151> 2005-04-29
<160> 7
<170> PatentIn version 3.1
<210> 1
   <211> 158
   <212> DNA
   <213> Pseudomonas aeruginosa
<400> 1
   gcggtgccgt tgacggcagc ctgagcgacg aagccgctct gcgtgcgatc accaccttct 60
   acttcgagta cgacagctcc gacctgaagc cggaagccat gcgcgctctg gacgtacacg 120
   cgaaagacct gaaaggcagc ggtcagcgcg tagtgctg 158
<210> 2
   <211> 19
   <212> DNA
   <213> Artificial sequence
<220>
   <223> primer F3 for detecting Pseudomonas aeruginosa using the LAMP amp lification technique according to the invention
<400> 2
   cctccaaggg cggcgatgc 19
<210> 3
   <211> 23
   <212> DNA
   <213> Artificial sequence
<220>
   <223> primer B3 for detecting Pseudomonas aeruginosa using the LAMP amp lification technique according to the invention
<400> 3
   tgcctttcag gtctttcgcg tgt 23
<210> 4
   <211> 43
   <212> DNA
   <213> Artificial sequence
<220>
   <223> primer FIP for detecting Pseudomonas aeruginosa using the LAMP am plification technique according to the invention
<400> 4
   ctgccgtcaa cggcaccgct tttccggtga aggtgccaat ggc 43
<210> 5
   <211> 45
   <212> DNA
   <213> Artificial sequence
<220>
   <223> primer BIP for detecting Pseudomonas aeruginosa using the LAMP am plification technique according to the invention
<400> 5
   gcgtgcgatc accaccttct acttttcaga gcgcgcatgg cttcc 45
<210> 6
   <211> 19
   <212> DNA
   <213> Artificial sequence
<220>
   <223> primer Loop F for detecting Pseudomonas aeruginosa using the LAMP amplification technique according to the invention
<400> 6
   cctgcgttcg ggtcgacgc 19
<210> 7
   <211> 19
   <212> DNA
   <213> Artificial sequence
<220>
   <223> primer LoopB for detecting Pseudomonas aeruginosa using the LAMP amplification technique according to the invention
<400> 7
   cgagtacgac agctccgacc 20